(19) Europäisches Patentamt
European Patent Office
Office européen des brevets

(11) Veröffentlichungsnummer: **0 397 042 B1**

(12) **EUROPÄISCHE PATENTSCHRIFT**

(45) Veröffentlichungstag der Patentschrift: **16.08.95**

(51) Int. Cl.[6]: **C07D 217/06**, C07D 217/20

(21) Anmeldenummer: **90108415.2**

(22) Anmeldetag: **04.05.90**

# (54) Herstellung von Isochinolinderivaten.

(30) Priorität: **10.05.89 CH 1751/89**
**16.03.90 CH 864/90**

(43) Veröffentlichungstag der Anmeldung:
**14.11.90 Patentblatt 90/46**

(45) Bekanntmachung des Hinweises auf die Patenterteilung:
**16.08.95 Patentblatt 95/33**

(84) Benannte Vertragsstaaten:
**AT BE CH DE DK FR GB IT LI NL**

(56) Entgegenhaltungen:
**EP-A- 0 176 856**
**EP-A- 0 307 168**
**EP-A- 0 315 886**

**CHEMICAL ABSTRACTS, Band 111, Nr. 15, 9. Oktober 1989, Seite 805,Zusammenfassung Nr. 134625b, Columbus, Ohio, US; & JP-A-01 34 964 (TAKASAGO PERFUMERY) 06-02-1989**

(73) Patentinhaber: **F. HOFFMANN-LA ROCHE AG**
**Postfach 3255**
**CH-4002 Basel (CH)**

(72) Erfinder: **Broger, Emil Albin, Dr.**
**Obere Egg 11**
**CH-4312 Magden (CH)**
Erfinder: **Heiser, Bernd, Dr.**
**Kalchmattweg 13A**
**D-7854 Inzlingen (DE)**

(74) Vertreter: **Cottong, Norbert A. et al**
**Grenzacherstrasse 124**
**Postfach 3255**
**CH-4002 Basel (CH)**

**EP 0 397 042 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein neues Verfahren zur Herstellung von Isochinolinderivaten, welche wertvolle Zwischenprodukte in der Synthese von Dextromethorphan darstellen.

Das erfindungsgemässe Verfahren ist dadurch gekennzeichnet, dass man ein Isochinolinderivat der allgemeinen Formel

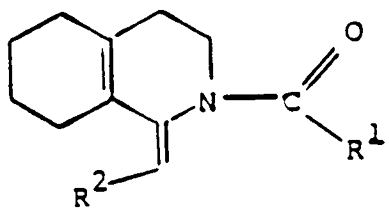

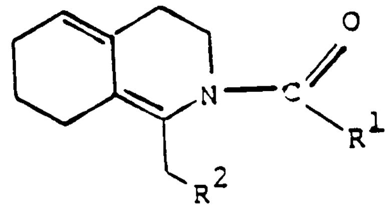

oder

E-Form II

III

oder ein Gemisch von Isochinolinderivaten der allgemeinen Formeln

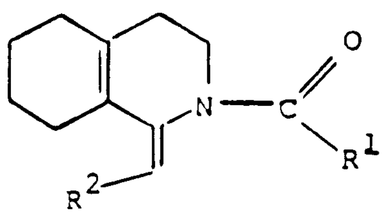

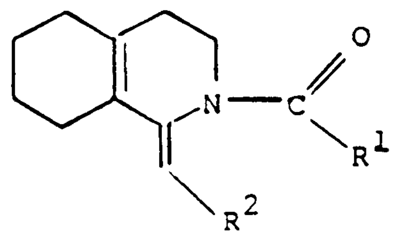

E-Form II

Z-Form I

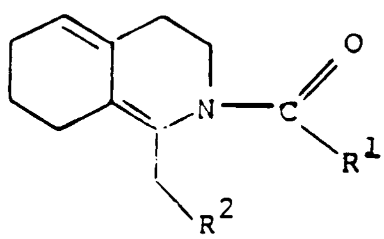

III

worin $R^1$ niederes Alkyl, Aryl oder Aryl-niederes Alkyl und $R^2$ Phenyl oder p-Methoxy-substituiertes Phenyl bedeuten,

isomerisiert, und dass man, gewünschtenfalls, ein so erhaltenes Isochinolinderivat der allgemeinen Formel in der Z-Form

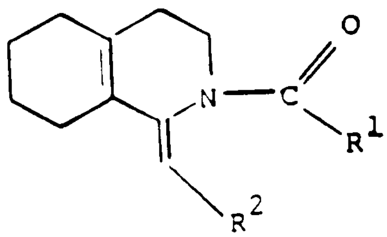

I

Z-Form

worin $R^1$ und $R^2$ die obige Bedeutung haben, asymmetrisch hydriert zu einer Verbindung der allgemeinen Formel

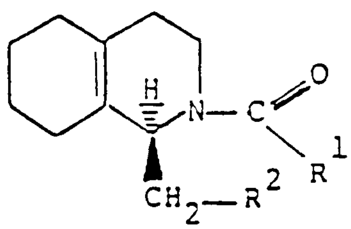

IV

worin $R^1$ und $R^2$ obige Bedeutung haben.

In C.A. 111: 134625b und in den europäischen Patentanmeldungen EP-A-0 307 168 und EP-A-0 315 886 werden bereits asymmetrische Hydrierverfahren zur Herstellung von gewissen Isochinolinderivaten beschrieben, wobei jedoch stets erwähnt wird, dass nur ein in der (Z)-Form vorliegendes Ausgangsprodukt eingesetzt werden kann.

Im Rahmen der vorliegenden Erfindung bedeutet der Ausdruck "niederes Alkyl" geradkettige oder verzweigte Alkylgruppen mit 1 bis 8 Kohlenstoffatomen, wie z.B. Methyl, Aethyl, n-Propyl, Isopropyl, Butyl, tert. Butyl, Pentyl, Hexyl, Heptyl, Octyl und dergleichen. Der Ausdruck "Aryl" bedeutet unsubstituiertes oder substituiertes Phenyl oder Naphthyl. Der Ausdruck Aryl-niederes Alkyl bedeutet Gruppen, in denen der Arylrest die vorhergehende Bedeutung hat und der niedere Alkylrest Gruppen mit 1 bis 3 Kohlenstoffatomen darstellt, wie z.B. Benzyl und dergleichen.

Im Rahmen der vorliegenden Erfindung bedeutet ferner das Zeichen "◄", dass sich der entsprechende Substituent oberhalb der Molekülebene befindet und das Zeichen "ıııı", dass sich der entsprechende Substituent unterhalb der Moleküleben befindet.

Im Zusammenhang mit den Liganden der Formeln VI und VII, können die erwähnten Phenyl- und Benzylreste sowohl unsubstituiert als auch in ortho, meta oder para Stellung oder auch mehrfach substituiert sein. Als Substituenten kommen hier in Frage niedere Alkyl- oder niedere Alkoxygruppen, vorzugsweise Methyl- oder Methoxygruppen, oder auch Di-niederes Alkylamino, vorzugsweise Dimethylaminogruppen, sowie Fluor. Der Ausdruck "niederes Alkyl" bedeutet in diesem Zusammenhang geradkettige oder verzweigte Alkylgruppen mit 1 bis 4 Kohlenstoffatomen wie Methyl, Aethyl, Propyl, Isopropyl, n-Buthyl, Isobutyl und tert. Butyl. Die Ausdrücke "niederes Alkoxy", Di-niederes Alkylamino" und "niederes Alkoxycarbonyl" bedeuten Gruppen, in denen der Alkylrest die vorhergehende Bedeutung hat. Als Schutzgruppen für die Hydroxymethylgruppe kommen im Rahmen der vorliegenden Erfindung insbesondere in Frage die üblichen, Aether-bildenden Gruppen wie z. B. Benzyl, Methyl, tert. Butyl, Methoxymethyl und dergleichen, sowie auch Ester-bildende Gruppen wie etwa Acetyl, Benzoyl und dergleichen.

Die Isomerisierung einer Verbindung der Formeln II (E-Form) oder III, oder eines Gemisches von Isochinolinderivaten der Formeln II (E-Form), I (Z-Form) und III, kann sowohl durch Erhitzen in einem chlorierten Kohlenwasserstoff oder in einem, einen chlorierten Kohlenwasserstoff enthaltenden Lösungsmittel, sowie auch katalytisch durchgeführt werden.

Das Erhitzen in einem chlorierten Kohlenwasserstoff oder in einem, einen chlorierten Kohlenwasserstoff enthaltenden Lösungsmittel kann bei einer Temperatur von etwa 50° C bis etwa 200° C, vorzugsweise von etwa Rückflusstemperatur bis etwa 150° C, und notfalls unter Druck, erfolgen. Als chlorierte Kohlenwasserstoffe können diejenigen verwendet werden, welche gewöhnlich als Lösungsmittel verwendet werden, wie beispielsweise Methylenchlorid, Chloroform, 1,2-Dichloräthan und dergleichen. Als Lösungsmittel können in diesem Zusammenhang insbesondere niedere Alkanole mit 1 bis 5 Kohlenstoffatomen verwendet werden, wie etwa Methanol, Aethanol, Propanol und dergleichen. Ein bevorzugtes, einen chlorierten Kohlenwasserstoff enthaltendes Lösungsmittel ist Methylenchlorid enthaltendes Methanol.

Die katalytische Isomerisierung kann in einem inerten organischen Lösungsmittel bei einer Temperatur von etwa -10° C bis etwa 150° C durchgeführt werden, notfalls unter Inertgasdruck. Vorzugsweise erfolgt diese Isomerisierung jedoch bei einer Temperatur von etwa Raumtemperatur bis etwa 120° C. Als inerte, organische Lösungsmittel können hier verwendet werden, aromatische Kohlenwasserstoffe, wie beispielsweise Benzol oder Toluol, Alkohole mit 1-3 C-Atomen wie Methanol, Aethanol oder Propanol, Aether wie Terahydrofuran oder Dioxan oder auch chlorierte Kohlenwasserstoffe wie etwa Methylenchlorid, Chloroform, 1,2-Dichloraethan und dergleichen. Weiterhin können Gemische dieser Lösungsmittel verwendet werden. Als Katalysatoren für diese katalytische Isomerisierung können gewöhnliche Isomerisierungskatalysatoren verwendet werden. Als Beilspiele für derartige Katalysatoren können genannt werden, Säuren wie etwa Halogenwasserstoffe, z.B. Chlorwasserstoff oder Bromwasserstoff, Sulfonsäuren, z.B. p-Toluolsulfonsäure, Sulfinsäuren, z.B. Phenylsulfinsäure, Schwefelsäure, Salpetersäure, Lewis-Säuren, z.B. $BF_4$, $TiCl_4$ usw., oder auch Carbonsäuren wie z.B. Essigsäure oder Propionsäure und dergleichen. Weiterhin können genannt werden, Jod, 2,2,6,6-Tetramethylpiperidyloxyl oder auch Uebergangsmetallkomplexe, z.B. Rhodiumkomplexe wie etwa $RhCl_3$-Hydrat, Rutheniumkomplexe wie etwa $RuCl_3$-Hydrat oder auch Palladiumkomplexe wie etwa $PdCl_2(CH_3CN)_2$ und dergleichen.

Durch die asymmetrische Hydrierung der Isochinolinderivate der Formel I in der Z-Form, erhält man diejenigen der Formel

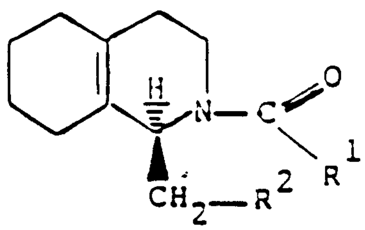

IV

worin $R^1$ und $R^2$ obige Bedeutung haben.

Diese asymmetrische Hydrierung kann in an sich bekannter Weise durchgeführt werden. Insbesondere kann diese Hydrierung in Gegenwart eines Rutheniumkatalysators der Formel V oder Va durchgeführt werden:

$Ru(Z)_2L$ V

oder

$[Ru\ (Z^2)_{2-n}(L)(X)]Z^3_n$ Va

worin Z Halogen oder einen Rest der Formel A-C00⁻ darstellt A niederes Alkyl, Aryl, halogeniertes niederes Alkyl oder halogeniertes Aryl bedeutet, $Z^2$ Halogen, X Benzol, Hexamethylbenzol oder p-Cymol und $Z^3$ Halogen, $BF_4$, $ClO_4$ oder $B(Phenyl)_4$ und n die Zahl 1 oder 2 bedeuten und L einen Liganden der Formel VI oder VII darstellt:

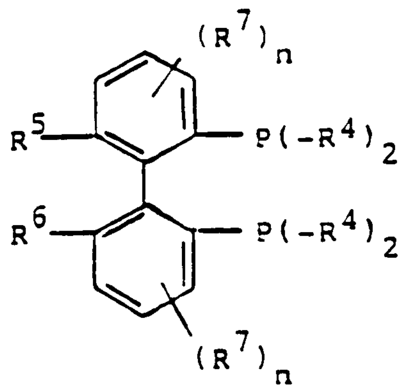

VI

worin $R^4$ Phenyl, $R^5$ und $R^6$, welche gleich oder verschieden sein können, Wasserstoff, niederes Alkyl, niederes Alkoxy, Di-niederes Alkylamino, geschütztes Hydroxymethyl oder $R^5$ und $R^6$ zusammen die Gruppen $(-CH_2-)_m$, $-CH_2-O-CH_2-$,

$(-CH_2-)_m$, $-CH_2-O-CH_2-$, 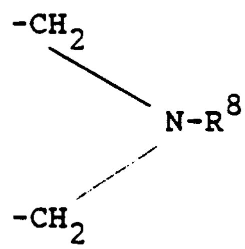 oder 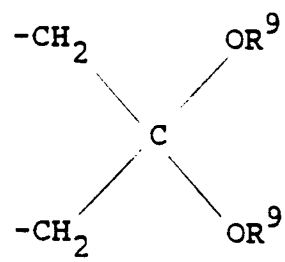

bedeuten, wobei m eine Zahl 3 bis 5, $R^8$ niederes Alkyl, Phenyl oder Benzyl und $R^9$ niederes Alkyl oder beide $R^9$ zusammen Di- oder Trimethylen darstellen, $R^7$ Methyl, niederes Alkoxy, Di-niederes Alkylamino oder Fluor und n die Zahl 0, 1, 2 oder 3 bedeuten;

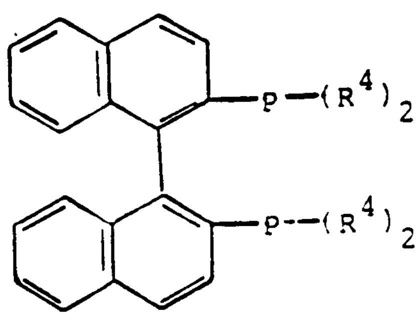

VII

worin $R^4$ die obige Bedeutung hat, und die Naphthalinringe gegebenenfalls noch in ortho-Stellung mit Methyl, Aethyl, Halogen, Di-niederes Alkylamino oder niederes Alkoxy substituiert sind.

Der Ausdruck Halogen bedeutet im Rahmen der vorliegenden Erfindung Fluor, Chlor, Brom oder Jod.

Der Ausdruck "halogeniertes niederes Alkyl" bedeutet im Rahmen der vorliegenden Erfindung niedere Alkylgruppen mit einer variablen Anzahl von Halogenatomen, insbesondere Chlor oder Fluor, wobei sich vorzugsweise ein Halogenatom in $\alpha$-Stellung zur $-COO^-$ Gruppe befindet.

Bevorzugte halogenierte niedere Alkylgruppen sind perfluorierte und perchlorierte niedere Alkylgruppen, beispielsweise Trifluormethyl , Pentafluoraethyl und dergleichen. Halogen bedeutet Fluor, Chlor, Brom oder Jod, insbesondere Fluor oder Chlor. Der Ausdruck "halogeniertes Aryl" bedeutet vorzugsweise Perfluorphenyl oder Perfluorbiphenyl.

Bevorzugte Liganden sind diejenigen der Formel VI. Von diesen wiederum sind diejenigen bevorzugt, worin $R^4$ unsubstituiertes oder Methyl-substituiertes Phenyl, $R^5$ und $R^6$ gleich sind und niederes Alkyl oder zusammen die Gruppe $-CH_2-O-CH_2-$, n die Zahl 0 oder 1 und $R^7$ Methyl, Fluor oder Di-niederes Alkylamino bedeuten. Falls n die Zahl 1 bedeutet, befindet sich der Substituent $R^7$ vorzugsweise in meta-Stellung zum

Phosphoratom.

Als Beispiele von besonders bevorzugten Liganden der Formel VI können die folgenden genannt werden:

(S)-(6,6′-Dimethyl-2,2′-biphenylylen)bis(diphenylphosphin);

(S)-(6,6′-Dimethyl-2,2′-biphenylylen)bis(di-p-tolylphosphin).

Die Liganden der Formeln VI und VII sind bekannte Verbindungen, z.B. aus der europäischen Publikation No. 104 375 bzw. aus der japanischen Patentanmeldung No. 136 605/1978.

Die Rutheniumkatalysatoren der Formel V können in an sich bekannter Weise hergestellt werden. Sie können beispielsweise dadurch erhalten werden, dass man einen Rutheniumkomplex der Formel

$[Ru\ (Z^1)_2L^1{}_m]_n \bullet (H_2 0)_p$ VIII

worin $Z^1$ Halogen oder einen Rest der Formel $A^1$-$C00^-$ darstellen, $A^1$ halogeniertes niederes Alkyl, $L^1$ einen neutralen Liganden, m die Zahl 0,1,2 oder 3, n die Zahl 1 oder 2 und p die Zahl 0 oder 1 darstellen, mit einem chiralen Diphosphinliganden der Formel VI oder VII umsetzt, oder, dass man einen Rutheniumkomplex der Formel

$Ru\ (CF_3C00)_2\ L$ IX

worin L obige Bedeutung hat,

mit einem, das Anion Z enthaltendes Salz umsetzt, worin Z obige Bedeutung hat.

Die Rutheniumkatalysatoren der Formel Va können ebenfalls in an sich bekannter Weise hergestellt werden, beispielsweise in Analogie zu dem Verfahren gemäss K. Mashima et al., J. Chem. Soc. Chem. Commun.1989, 1208-1210.

Der Ausdruck "neutraler Ligand" bedeutet im Rahmen der vorliegenden Erfindung einen leicht austauschbaren Liganden, wie etwa ein Diolefin, z.B. Norbornadien, 1,5-Cyclooctadien usw., oder ein Nitril, z.B. Acetonitril, Benzonitril usw. Falls m die Zahl 2 oder 3 bedeutet, können die Liganden gleich oder verschieden sein.

Die Rutheniumkomplexe der Formel VIII, welche als Ausgangsmaterial verwendet werden, sind bekannte Substanzen oder Analoge bekannter Substanzen, welche leicht in zur Herstellung der bekannten analoger Weise erhalten werden können, beispielsweise gemäss Albers, M.0. et al., J. Organomet. Chem., 272 (1984) C62 - C66.

Die Umsetzung eines Rutheniumkomplexes der Formel VIII mit einem chiralen Diphosphinliganden der Formel VI oder VII kann in an sich bekannter Weise durchgeführt werden. Diese Reaktion kann zweckmässig in einem inerten organischen Lösungsmittel erfolgen. Als Beispiele derartiger Lösungsmittel können genannt werden, z.B. Aether wie Tetrahydrofuran oder Dioxan, Ketone wie etwa Aceton, niedere Alkohole wie etwa Methanol, Aethanol usw., halogenierte Kohlenwasserstoffe wie Methylenchlorid, Chloroform und dergleichen, oder auch Gemische derartiger Lösungsmittel. Die Reaktion kann zudem bei einer Temperatur zwischen etwa 0° C und etwa 100° C und vorzugsweise zwischen etwa 15° C und etwa 60° C erfolgen, jedoch unter striktem Ausschluss von Sauerstoff.

Die Umsetzung eines Rutheniumkomplexes der Formel IX mit einem das Anion Z enthaltenden Salz, kann in an sich bekannter Weise erfolgen. Der Ausdruck "ein das Anion Z enthaltendes Salz" bedeutet im Rahmen der vorliegenden Erfindung beispielsweise Ammoniumsalze, Alkalimetallsalze oder andere geeignete Metallsalze. Um die Löslichkeit derartiger Salze zu verbessern, können in gewissen Fällen auch Kronenäther zugesetzt werden.

Bei der Durchführung der vorhergehend erwähnten asymmetrischen Hydrierungen, können die Komplexe der Formel V zuerst hergestellt werden und dann einer Lösung der zu hydrierenden Substanz zugegeben werden. Alternativ können sie jedoch auch in situ, in Gegenwart oder Abwesenheit einer zu hydrierenden Substanz hergestellt werden.

Die asymmetrische Hydrierung kann in einem geeigneten, unter den Reaktionsbedingungen inerten organischen Lösungsmittel erfolgen. Als derartige Lösungsmittel können insbesondere genannt werden, niedere Alkohole wie z.B. Methanol oder Aethanol oder Gemische derartiger Alkohole mit halogenierten Kohlenwasserstoffen wie Methylenchlorid, Chloroform und dergleichen, oder mit cyklischen Aether wie Tetrahydrofuran oder Dioxan und dergleichen. Das Verhältnis von Ruthenium zu Ligand L liegt zweckmässig zwischen etwa 0,5 und etwa 2 Mol, vorzugsweise bei etwa 1 Mol Ruthenium pro Mol Ligand. Das Verhältnis von Ruthenium, in den Komplexen der Formel V oder Va, zu den zu hydrierenden Substanzen liegt zweckmässig zwischen etwa 0,005 und etwa 2 Mol %, vorzugsweise zwischen etwa 0,01 und etwa 0,1 Mol %. Die asymmetrische Hydrierung mit den Komplexen der Formel V oder Va erfolgt zweckmässig bei

einer Temperatur von etwa 50° C bis etwa 200° C, vorzugsweise von etwa 80° C bis etwa 160° C. Diese Hydrierung erfolgt auch zweckmässig unter Druck, vorzugsweise bei einem Druck von etwa 5 bis etwa 200 bar, vorzugsweise von etwa 20 bis etwa 100 bar.

Die in dem erfindungsgemässen Verfahren als Ausgangsmaterialien verwendeten Verbindungen der Formel II in der (E)-Form, sowie auch die Gemische der Verbindungen der Formel II, in der (E)-Form, der Formel I, in der (Z)-Form, und der Formel III, können in an sich bekannter Weise hergestellt werden. So können die erwähnten Gemische beispielsweise hergestellt werden durch Umsetzung einer Verbindung der Formel

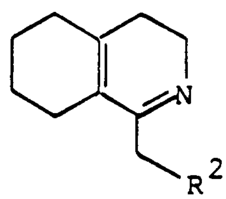

X

worin $R^2$ obige Bedeutung hat,
mit einem Acylierungsmittel der Formel

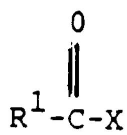

XI

worin $R^1$ obige Bedeutung hat und X Halogen oder einen Rest der Formel

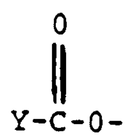

bedeutet, worin Y niederes Alkyl darstellt.

Die Acylierung einer Verbindung der Formel X mit einem Acylierungsmittel der Formel XI kann in einem inerten organischen Lösungsmittel in Gegenwart einer Base und bei einer Temperatur von etwa -20° C bis etwa 50° C, vorzugsweise von etwa 0° C bis etwa 20° C erfolgen. Geeigente Lösungsmittel sind aprotische Lösungsmittel wie aliphatische oder aromatische Kohlenwasserstoffe, z.B. Hexan, Benzol, Toluol usw., oder auch Aether wie z.B. Diäthyläther, Tetrahydrofuran, Dioxan usw.. Als Basen können hier verwendet werden, Amine wie z.B. Triäthylamin oder Pyridin, oder auch Alkali- und Erdalkalimetallsalze organischer Säuren, wie z.B Natriumformiat, Natriumacetat und dergleichen. Aus so erhaltenen Gemischen der Verbindungen II (E-Form), I (Z-Form) und III, können diejenigen der Formel II in der E-Form leicht z.B. durch Säulenchromatographie isoliert werden.

Die in dem erfindungsgemässen Verfahren ebenfalls als Ausgangsmaterial verwendeten Verbindungen der Formel III können z.B. durch Isomerisierung von Verbindungen der Formel II, in der E-Form, erhalten werden. Diese Isomerisierung kann beispielsweise in zur vorhergehend erwähnten Isomerisierung analoger Weise durchgeführt werden. Hierbei wird der Reaktionsverlauf z.B. gaschromatographisch verfolgt und die Reaktion bei der maximalen Konzentration an Verbindung III abgebrochen.

Die folgenden Beispiele dienen zur Illustrierung der Erfindung und stellen in keiner Weise irgend eine Beschränkung dar. In diesen Beispielen haben die gewählten Abkürzungen folgende Bedeutung:

| | |
|---|---|
| GC | : Gaschromatographie |
| THF | : Tetrahydrofuran |
| BIPHEMP | : (S)-(6,6'-Dimethyl-biphenylylen)bis(diphenylphosphin) |
| COD | : 1,5-Cyclooctadien |

Beispiel 1

Eine Lösung von 0,2 g (E)-2-Acetyl-1,2,3,4,5,6,7,8-octahydro-1-(p-methoxybenzyliden)isochinolin und 8,7 mg Dichloro bis (acetonitril) palladium(II) in 10 ml trockenem Tetrahydrofuran wurde während 7 Stunden unter Argon am Rückfluss erhitzt. Die gelbe Lösung wurde bei 15 mbar eingedampft und der Rückstand in Diaethylaether gelöst. Zur Abtrennung des Katalysators wurde die Aetherlösung durch ein kurzes Kieselgelpolster filtriert. Nach Eindampfen des Filtrates erhielt man 0,19 g (Z)-2-Acetyl-1,2,3,4,5,6,7,8-octahydro-1-(p-methoxybenzyliden)isochinolin, mit einem Smp. von 101-102° C; Gehalt gemäss GC 96 %:

Das als Ausgangsmaterial verwendete (E)-2-Acetyl-1,2,3,4,5,6,7,8-octahydro-1-(p-methoxybenzyliden)-isochinolin wurde wie folgt hergestellt:
Zu einer gerührten Lösung von 0,80 Mol rohem 1-(p-Methoxybenzyl)-3,4,5,6,7,8-hexahydroisochinolin [erhalten gem. O. Schnider et al., Helv. Chim. Acta 33, 1437 (1950)] in 2 Liter Toluol wurden bei 0-3° C unter Argon während 20 Minuten 164 g (1,61 Mol) Essigsäureanhydrid gegeben. Anschliessend werden während 30 Minuten 204 g (2,01 Mol) Triäthylamin zugesetzt. Das Gemisch wurde über Nacht gerührt und die Temperatur auf 15°-20° C ansteigen gelassen. Nach Abkühlen auf 5° C wurde die Reaktionsmischung nacheinander mit Eiswasser, 2N HCl, Eiswasser, 2N Na0H, Eiswasser und gesättigter Sole gewaschen. Die organische Phase wurde über $Na_2S0_4$ getrocknet und dann eingeengt, wobei man 205 g eines rotbraunen Oeles erhielt. Nach Kolonnenchromatographie und Kristallisation aus Methanol erhielt man 41,3 g, gemäss Dünnschicht- und Gaschromatographie, reines (E)-2-Acetyl-1,2,3,4,5,6, 7,8-octahydro-1-(p-methoxybenzyliden)isochinolin. Smp. 74,5° - 76° C.

Beispiel 2

In zu Beispiel 1 analoger Weise wurden die in der Tabelle 1 aufgeführten Isomerisierungen von (E)-2-Acetyl-1,2,3,4,5, 6,7,8-octahydro-1-(p-methoxybenzyliden)isochinolin durchgeführt:

Tabelle 1

| Katalysator 5 Mol-% | Lösungsmittel c = 2 % | T (°C) | Zeit (h) | Produkt | |
|---|---|---|---|---|---|
| | | | | % (Z) | % (E) |
| HBr | Methanol | 65 | 7 | 78 | < 1 |
| p-Toluolsulfonsäure | Toluol | 111 | 16 | 89 | < 1 |
| p-Toluolsulfonsäure | Methanol | 65 | 16 | 95 | < 1 |
| Phenylsulfinsäure | Methanol | 65 | 16 | 81 | 7 |
| Keiner | Essigsäure | 118 | 7 | 85 | 7 |
| Keiner | $CH_2Cl_2$ | 100 a) | 22 | 91 | < 1 |
| BF3-Aetherat | Benzol | 80 | 7 | 95 | < 1 |
| $TiCl_4$ | THF | 66 | 6 | 92 | < 1 |
| $PdCl_2(CH_3CN)_2$ | $CH_2Cl_2$ | 25 | 16 | 94 | < 1 |
| $PdCl_2(CH_3CN)_2$ | THF | 66 | 16 | 96 | < 1 |
| $RhCl_3$-Hydrat | THF | 66 | 7 | 96 | < 1 |
| RuC13-Hydrat | THF | 66 | 7 | 89 | < 1 |
| Jod | $CH_2Cl_2$ | 41 | 7 | 89 | < 1 |
| Jod | Toluol | 111 | 16 | 88 | < 1 |
| TEMPO b) | Toluol | 111 | 16 | 82 | < 1 |

a) 20 bar Argon
b) 2,2,6,6-Tetramethyl-1-piperidinyloxyl

Beispiel 3

Eine Lösung von 0,2 g 2-Acetyl-1-(p-methoxybenzyl)-2,3,4,6,7,8-hexahydroisochinolin und 2,5 mg Acetylchlorid in 10 ml trockenem Methanol wurde 16 Stunden unter Argon am Rückfluss erhitzt. Das Reaktionsgemisch wurde eingedampft und der Rückstand in Diaethylaether gelöst. Die Aetherlösung wurde mit gesättigter Natriumbicarbonatlösung, Wasser und gesättigter Kochsalzlösung gewaschen, über Natriumsulfat getrocknet und am Rotationsverdampfer im Vakuum eingedampft. Der Rückstand bestand

gemäss GC aus 95 % (Z)-2-Acetyl-1,2,3,4,5,6,7,8-octahydro-1-(p-methoxybenzyliden)isochinolin. Umkristallisation aus Methanol lieferte 140 mg reines (Z)-Isomeres: Smp. 101-102° C.

Das als Ausgangsmaterial verwendete 2-Acetyl-1-(p-Methoxybenzyl)-2,3,4,6,7,8-hexahydroisochinolin wurde wie folgt hergestellt:

Eine Lösung von 2,1 g (E)-2-Acetyl-1,2,3,4,5,6,7,8-octahydro-1-(p-methoxybenzyliden)isochinolin (hergestellt gemäss Beispiel 1) in 120 ml Methylenchlorid wurde während 2 Stunden unter Argon am Rückfluss erhitzt. Das Lösungsmittel wurde dann bei 30°C/20 mbar abdestilliert. Der Rückstand, 2,3 g eines farblosen Oeles, enthielt gemäss GC 61% des gewünschten Produktes. Säulenchromatographie und Kristallisation aus Isopropyläther ergaben 700 mg, gemäss Dünnschicht- und Gaschromatographie, reines 2-Acetyl-1-(p-methoxybenzyl)-2,3,4,6,7,8-hexahydroisochinolin als weisse Kristalle. Smp. 48°-50° C.

Beispiel 4

Zu einer Lösung von 98,1 g (0,330 Mol) eines Gemisches von (E)-2-Acetyl-1,2,3,4,5,6,7,8-octahydro-1-(p-methoxybenzyliden)isochinolin, (Z)-2-Acetyl-1,2,3,4,5,6,7,8-octahydro-1-(p-methoxybenzyliden)isochinolin und 2-Acetyl-1-(p-methoxybenzyl)-2,3,4,6,7,8-hexahydroisochinolin in 2 Liter Methylenchlorid wurde unter Argon tropfenweise eine Lösung von 8,4 g Jod (0,033 Mol) in 400 ml Methylenchlorid zugegeben. Nach Rühren der entstandenen roten Lösung über Nacht bei Raumtemperatur, wurde diese mit 1 x 250 ml einer 0,5 N Natriumthiosulfatlösung und 1 x mit 250 ml Sole gewaschen. Die wässrigen Extrakte wurden mit den gleichen 2 x 250 ml Methylenchlorid zurückgewaschen. Die vereinigten organischen Extrakte wurden über Natriumsulfat getrocknet und am Rotationsverdampfer eingeengt, wobei man 98,9 g eines roten Oeles erhielt. Dieses Oel enthielt gemäss GC 93,3 % (Z)-2-Acetyl-1,2,3,4,5,6,7,8-octahydro-1-(p-methoxybenzyliden)isochinolin.

Dieses rohe Z-Isomere wurde unter Rühren mit 650 ml Diisopropylaether am Rückfluss erhitzt, bis, mit Ausnahme von wenig schwarzem Rückstand, sämtliches Material gelöst war. Die heisse Lösung wurde abdekantiert und heiss gehalten. Der Rückstand wurde während einer Stunde mit 300 ml Diisopropylaether extrahiert und die Lösung dekantiert. Die heissen Lösungen wurden vereint und mit 2 g Aktivkohle während 15 Minuten gerührt. Nach Filtration wurde die gelbliche Lösung bei 40° C unter vermindertem Druck auf ein Volumen von ca. 500 ml eingeengt. Die Kristallisation des Produktes, welche bereits während der Destillation eingesetzt hat, wurde bei 0° C über Nacht fortgesetzt. Die Kristalle wurden dann abfiltriert, mit 2 x 60 ml kaltem (-20° C) Diisopropylaether gewaschen und bei 30° C/0,01 mbar getrocknet. Man erhielt 68,8 g (Z)-2-Acetyl-1,2,3,4,5,6,7,8-octahydro-1-(p-methoxybenzyliden)isochinolin als weisse Kristalle mit einem Schmelzpunkt von 100° - 102° C.

Das als Ausgangsmaterial verwendete Isomerengemisch wurde wie folgt hergestellt:

In einem 4,5 L Vierhalskolben, versehen mit einem Thermometer, einem 500 ml Tropftrichter, einem mechanischen Rührer und einem Gaseinlassrohr, wurden 81,5 g Triaethylamin (0,805 Mol) und 2 L Toluol vorgegeben und die Lösung auf 0° - 2° C abgekühlt. Dann wurden unter Stickstoff und bei 0° - 3° C nacheinander zunächst während 5 Minuten 500 ml einer Lösung von 0,8 Mol 1-(p-Methoxybenzyl)-3,4,5,6,7,8-hexahydroisochinolin-Hydrochlorid (hergestellt gem. O. Schnider et al., Helv. Chim. Acta 33, 1437 (1950)) in Toluol zugegeben, dann während 15 Minuten 164,4 g (1,610 Mol) Essigsäureanhydrid und hierauf während 15 Minuten 203,6 g (2,012 Mol) Triaethylamin. Das Eisbad wurde anschliessend entfernt und die gelbe Lösung während 20 Stunden bei 20° C gerührt. Das Reaktionsgemisch wurde auf 2° C abgekühlt und nacheinander mit folgenden kalten Lösungen extrahiert:

1 x mit 1 L und 2 x mit 0,5 L Eiswasser,
1 x mit 200 ml und 2 x mit 100 ml 2N Natriumhydroxid-Lösung,
1 x mit 0,5 L Eiswasser,
1 x mit 0,5 L und 2 x mit 0,3 L 2N Chlorwasserstoffsäure,
3 x mit 0,5 L Wasser und
1 x mit 0,5 L Sole.

Jeder wässrige Extrakt wurde mit den gleichen 2 x 0,3 L Portionen Toluol zurückgewaschen.

Die vereinten Toluollösungen wurden über Magnesiumsulfat getrocknet und bei 40° C/20 mbar eingedampft und man erhielt 234,1 g (97,6 %) eines rotbraunen Oeles. Dieses bestand gemäss Dünnschichtchromatographie aus einem Gemisch von (E)-2-Acetyl-1,2,3,4,5,6,7,8-octahydro-1-(p-methoxybenzyliden)isochinolin, (Z)-2-Acetyl-1,2,3,4,5,6,7,8-octahydro-1-(p-methoxybenzyliden)isochinolin und 2-Acetyl-1-(p-methoxybenzyl)-2,3,4,6,7,8-hexahydroisochinolin im Verhältnis 40:45:15. Zur Entfernung ebenfalls noch vorhandener polarer Verunreinigungen wurde das rohe Material durch eine kurze Kolonne (Durchmesser 10 cm) von 1 Kg Kieselgel (0,04 - 0,063 mm) unter Verwendung von 7 Liter Benzol/Aethylacetat (3 : 1 v/v) als Eluiermittel filtriert. Durch Konzentration der Eluate erhielt man 196,2 g (81,9 %) eines rotbraunen Oeles,

welches gemäss Dünnschichtchromatographie nurmehr aus den erwähnten drei Komponenten bestand.

Beispiel 5

In einer Handschuhbox wurde ein 500 ml Autoklav mit 2 g (6,73 mMol) (Z)-2-Acetyl-1,2,3,4,5,6,7,8-octahydro-1-(p-methoxybenzyliden)isochinolin (hergestellt gemäss einem der Beispiele 1-4), 170 ml Methanol und 1,3 mg (0,0017 mMol; S/Ru = 4000) $Ru(CH_3C00)_2$(BIPHEMP) als Katalysator beladen. Die Hydrierung wurde bei 100° C und 35 bar während 24 Stunden durchgeführt. Die Hydrierlösung wurde eingedampft und der Rückstand in Diaethylaether gelöst. Nach Abtrennung des Katalysators über Kieselgel und Eindampfen des Filtrates erhielt man 1,98 g (S)-2-Acetyl-1-(p-methoxybenzyl)-1,2,3,4,5,6,7,8-octahydroisochinolin von 98,3 % e.e., welches aus Diisopropylaether umkristallisiert wurde.
$[\alpha]_D^{20}$ = + 53° (c=1 in $CH_3OH$); 100% e.e.; Smp. 80°-81,5° C.

Zur Bestimmung des e.e.-Wertes wurden die Produkte in einem Gemisch von Aethylenglycol und 40% wässriger Kaliumhydroxidlösung bei 170° C während 18 Stunden hydrolysiert. Das gebildete Amin wurde mit (-)-Camphansäurechlorid in Pyridin/4-Dimethylaminpyridin zum Gemisch der diastereomeren Amide umgesetzt, und letzteres mittels GC analysiert.

Beispiel 6

In einer Handschuhbox wurde ein 500 ml Autoklav mit 2,0 g, (6,73 mMol), (Z)-2-Acetyl-1-(p-methoxybenzyliden)-1,2,3,4,5,6,7,8-tetrahydroisochinolin, 140 ml Methanol, 28 ml Methylenchlorid und $Ru(CF_3COO)_2$(BIPHEMP) [hergestellt in situ aus 3,7 mg BIPHEMP und 3,0 mg $[Ru(CF_3C00)_2(COD)]_2H_2O$: E. Singleton et al. J. Organomet. Chem. 272 (1984) C62-C66] als Katalysator beladen. Die Hydrierung wurde bei 160° C und 60 bar während 1 Stunde durchgeführt, wonach der Umsatz 100 % betrug. Das Produkt wurde anlaog zu Beispiel 5 isoliert. Es wurden 1,9 g (S)-2-Acetyl-1-(p-methoxybenzyl)-1,2,3,4,5,6,7,8-octahydroisochinolin erhalten; 96,3 % e.e. Der e.e.-Wert wurde in zu Beispiel 5 analoger Weise bestimmt.

Beispiel 7

In einer Handschuhbox wurde ein 500 ml Autoklav mit 2,0 g, (6,7 mMol), (E)-2-Acetyl-1-(p-methoxybenzyliden)-1,2,3,4,5,6,7,8-tetrahydroisochinolin, 140 ml Methanol, 28 ml Methylenchlorid und $Ru(X)_2$-(BIPHEMP) als Katalysator beladen. Die Hydrierung wurde unter den in Tab. 2 angegebenen Bedingungen durchgeführt. Das Produkt, (S)-2-Acetyl-1-(p-methoxybenzyl)-1,2,3,4,5,6,7,8-octahydroisochinolin, wurde analog zu Beispiel 5 isoliert und analysiert. Der e.e.-Wert wurde in zu Beispiel 5 analoger Weise bestimmt.

Tabelle 2

| X | S/Ru | T (°C) | (bar) | Umsatz % | Zeit (h) | e.e. % (S) |
|---|---|---|---|---|---|---|
| $CH_3C00$ a) | 2000 | 100 | 60 | 95,4 | (5) | 93,8 |
| $CF_3C00$ | 2000 | 100 | 35 | 99,4 | (22) | 95,6 |
| Cl b) | 4000 | 100 | 35 | 90 | (42) | 97,3 |

a) Katalysator hergestellt in situ aus $Ru(CF_3C00)_2$BIPHEMP und 2 Moläquivalente Natriumacetat
b) Katalysator hergestellt in situ aus $Ru(CH_3C00)_2$BIPHEMP und 2 Moläquivalente HCl

Beispiel 8

In einer Glove-Box ($O_2$-Gehalt <1 ppm) wurde ein 500 ml-Autoklav mit 2.0 g (6.73 mMol) (Z)-2-Acetyl-1-(p-methoxybenzyliden)1,2,3,4,5,6,7,8-octahydroisochinolin, 140 ml Methanol und einer Katalysatorlösung von 1.53 mg [Ru{(S)-BIPHEMP}C1(p-Cymol)]BF4 (hergestellt in Analogie zu K. Mashima et al., J. Chem. Soc. Chem. Commun. 1989, 1208-1210) in 30 ml Methanol, beladen. Die Hydrierung erfolgte bei 100°C und 35 bar unter intensivem Rühren. Nach 18 Stunden betrug der Umsatz 99.4%. Die Hydrierlösung wurde analog zu Beispiel 5 aufgearbeitet. Man erhielt 1.94 g (S)-2-Acetyl-1-(p-methoxybenzyl)-1,2,3,4,5,6,7,8-octahydroisochinolin: e.e. 97.7 %.

## Patentansprüche

1. Verfahren zur Herstellung von Isochinolinderivaten, dadurch gekennzeichnet, dass man ein Isochinolinderivat der allgemeinen Formel

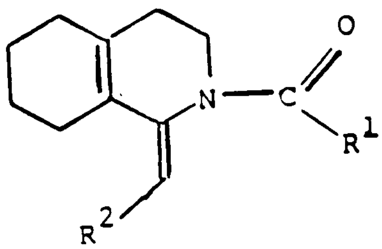

E-Form II

oder

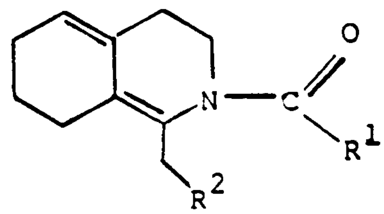

III

oder ein Gemisch von Isochinolinderivaten der allgemeinen Formeln

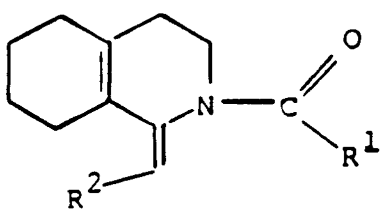

E-Form II

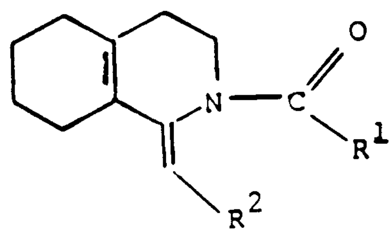

Z-Form I

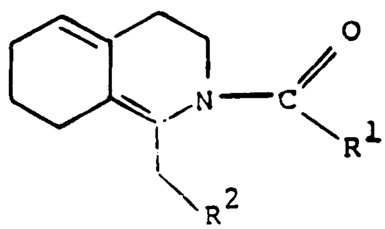

III

worin $R^1$ niederes Alkyl, Aryl oder Aryl-niederes Alkyl, und $R^2$ phenyl oder p-Methoxy-substituiertes Phenyl bedeuten,
isomerisiert, und dass man, gewünschtenfalls, ein so erhaltenes Isochinolinderivat der allgemeinen Formel in der Z-Form

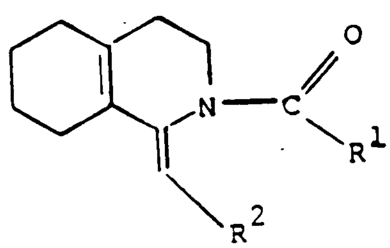

Z-Form I

worin $R^1$ und $R^2$ obige Bedeutung haben asymmetrisch hydriert zu einer Verbindung der allgemeinen Formel

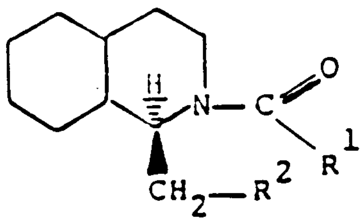

IV

worin $R^1$ und $R^2$ obige Bedeutung haben.

**2.** Verfahren gemäss Anspruch 1, dadurch gekennzeichnet, dass man die Isomerisierung einer Verbindung der Formel II in der E-Form, einer Verbindung der Formel III, oder eines Gemisches von Isochinolinderivaten der Formeln II (E-Form), I (Z-Form) und III durch Erhitzen in einem chlorierten Kohlenwasserstoff oder in einem, einen chlorierten Kohlenwasserstoff enthaltenden Lösungsmittel oder katalytisch durchführt.

**3.** Verfahren gemäss Anspruch 1 oder 2, dadurch gekennzeichnet, dass man die Isomerisierung durch Erhitzen auf eine Temperatur von etwa 50° C bis etwa 200° C, vorzugsweise von etwa Rückflusstemperatur bis etwa 150° C, durchführt.

**4.** Verfahren gemäss einem den Ansprüchen 1 bis 3, dadurch gekennzeichnet, dass man als chlorierten Kohlenwasserstoff bei der Isomerisierung Methylenchlorid, Chloroform oder 1,2-Dichloraethan verwendet.

**5.** Verfahren gemäss einem der Ansprüche 1 bis 4, dadurch gekennzeichnet, dass man als Lösungsmittel bei der Isomerisierung einen niederen Alkohol mit 1 bis 5 Kohlenstoffatomen verwendet.

**6.** Verfahren gemäss Anspruch 1, dadurch gekennzeichnet, dass man als Katalysator für die katalytische Isomerisierung eine Säure, insbesondere eine Halogenwasserstoffsäure, Jod, oder Uebergangsmetallkomplexe verwendet.

**7.** Verfahren gemäss Anspruch 1, dadurch gekennzeichnet, dass man die asymmetrische Hydrierung einer Verbindung der Formel I in der Z-Form, in Gegenwart eines Rutheniumkatalysators der Formel V oder Va durchgeführt

$Ru(Z)_2L$ V

oder

$[Ru\ (Z^2)_{2-n}(L)(X)]Z^3_n$ Va

worin Z Halogen oder einen Rest der Formel A-C00$^-$ darstellt, A niederes Alkyl, Aryl, halogeniertes niederes Alkyl oder halogeniertes Aryl bedeutet, $Z^2$ Halogen, X Benzol, Hexamethylbenzol oder p-Cymol und $Z^3$ Halogen, $BF_4$, $ClO_4$ oder B(Phenyl)$_4$ und n die Zahl 1 oder 2 bedeuten und L einen Liganden der Formel VI oder VII darstellt:

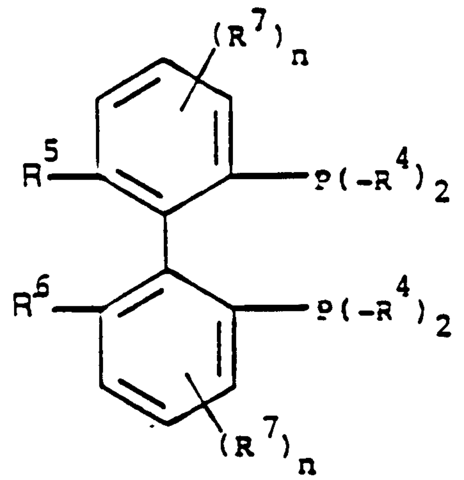

VI

worin $R^4$ Phenyl, $R^5$ und $R^6$, welche gleich oder verschieden sein können, Wasserstoff, niederes Alkyl, niederes Alkoxy, Di-niederes Alkylamino, geschütztes Hydroxymethyl oder $R^5$ und $R^6$ zusammen die Gruppen

$(-CH_2-)_m$, $-CH_2-O-CH_2-$, 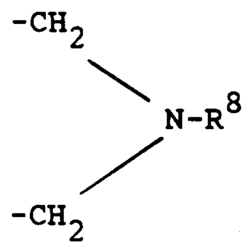 oder 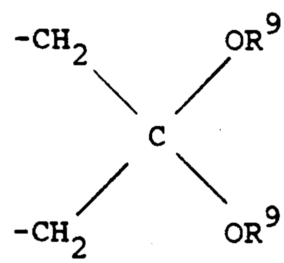

bedeuten, wobei m eine Zahl 3 bis 5, $R^8$ niederes Alkyl, Phenyl oder Benzyl und $R^9$ niederes Alkyl oder beide $R^9$ zusammen Di- oder Trimethylen darstellen, $R^7$ Methyl, niederes Alkoxy, Di-niederes Alkylamino oder Fluor und n die Zahl O, 1, 2 oder 3 bedeuten,

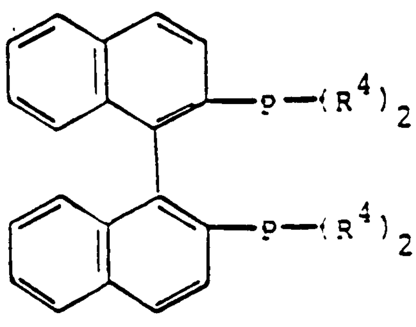

VII

worin $R^4$ die obige Bedeutung hat, und die Naphtalinringe gegebenenfalls noch in ortho-Stellung mit Methyl, Aethyl, Halogen, Di-niederes Alkylamino oder niederes Alkoxy substituiert sind.

**8.** Verfahren gemäss Anspruch 7, dadurch gekennzeichnet, dass man die asymmetrische Hydrierung bei einer Temperatur von etwa 50° C bis etwa 200° C, vorzugsweise von etwa 80° C bis etwa 160° C durchführt.

## Claims

**1.** A process for the manufacture of isoquinoline derivatives, characterized by isomerizing an isoquinoline derivative of the general formula

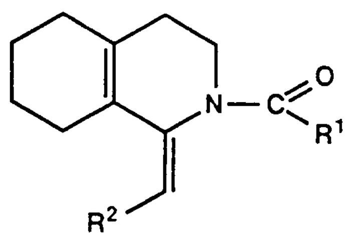

E-form II

or

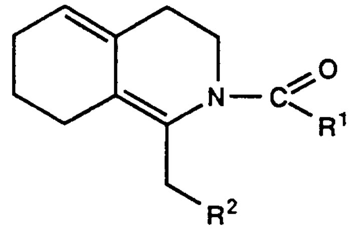

III

or a mixture of isoquinoline derivatives of the general formulae

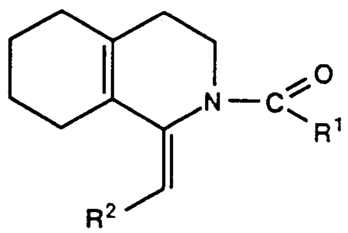

E-form II

or

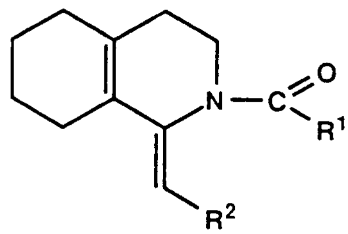

Z-form I

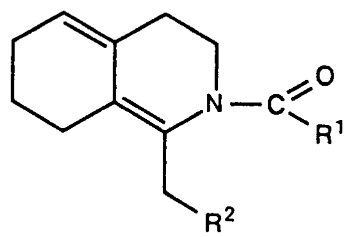

III

wherein $R^1$ signifies lower alkyl, aryl or aryl-lower alkyl and $R^2$ signifies phenyl or p-methoxy-substituted phenyl, and, if desired, asymmetrically hydrogenating a thus-obtained isoquinoline derivative of the general formula in the Z-form

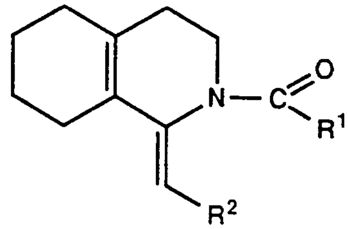

I

Z-form

wherein $R^1$ and $R^2$ have the above significance, to give a compound of the general formula

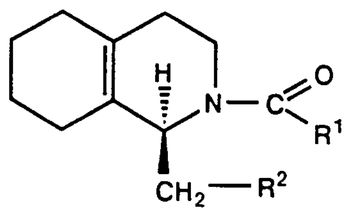

IV

wherein $R^1$ and $R^2$ have the above significance.

2. A process according to claim 1, characterized in that the isomerization of a compound of formula II in the E-form, of a compound of formula III or of a mixture of isoquinoline derivatives of formulae II (E-form), I (Z-form) and III is carried out by heating in a chlorinated hydrocarbon or in a solvent which contains a chlorinated hydrocarbon or catalytically.

3. A process according to claim 1 or 2, characterized in that the isomerization is carried out by heating to a temperature of about 50 °C to about 200 °C, preferably of about the reflux temperature to about 150 °C.

4. A process according to any one of claims 1 to 3, characterized in that methylene chloride, chloroform or 1,2-dichloroethane is used as the chlorinated hydrocarbon in the isomerization.

5. A process according to any one of claims 1 to 4, characterized in that a lower alcohol with 1 to 5 carbon atoms is used as the solvent in the isomerization.

6. A process according to claim 1, characterized in that an acid, especially a hydrohalic acid, iodine or a transition metal complex is used as the catalyst for the catalytic isomerization.

7. A process according to claim 1, characterized in that the asymmetric hydrogenation of a compound of formula I in the Z-form is carried out in the presence of a ruthenium catalyst of formula V or Va:

$Ru(Z)_2L$ V

or

$[Ru\ (Z^2)_{2-n}(L)(X)]Z^3_n$ Va

wherein Z signifies halogen or a residue of the formula A-COO-, A signifies lower alkyl, aryl, halogenated lower alkyl or halogenated aryl, $Z^2$ signifies halogen, X signifies benzene, hexamethylbenzene or p-cymene, $Z^3$ represents halogen, $BF_4$, $ClO_4$ or $B(phenyl)_4$, n signifies the number 1 or 2 and L signifies a ligand of formula VI or VII:

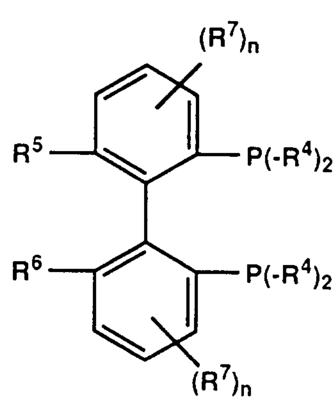

VI

wherein $R^4$ signifies phenyl, $R^5$ and $R^6$, which can be the same or different, signify hydrogen,

lower alkyl, lower alkoxy, di-lower alkylamino, protected hydroxymethyl or $R^5$ and $R^6$ together signify the group

$(-CH_2-)_m$, $-CH_2-O-CH_2-$, 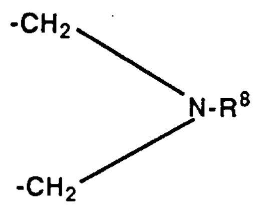 or 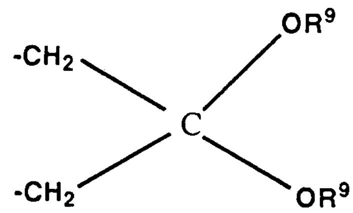

in which m signifies a number 3 to 5, $R^8$ signifies lower alkyl, phenyl or benzyl and $R^9$ signifies lower alkyl or both $R^9$'s together signify di- or trimethylene, $R^7$ signifies methyl, lower alkoxy, di-lower alkylamino or fluorine and n signifies the number 0, 1, 2 or 3,

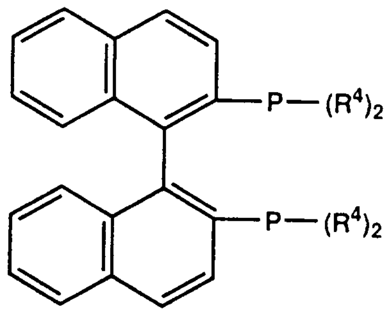

VII

wherein $R^4$ has the above significance and the naphthalene rings are optionally substituted in the ortho-position with methyl, ethyl, halogen, di-lower alkylamino or lower alkoxy.

8. A process according to claim 7, characterized in that the asymmetric hydrogenation is carried out at a temperature of about 50 °C to about 200 °C, preferably of about 80 °C to about 160 °C.

## Revendications

1. Procédé de préparation de dérivés d'isoquinoléine, caractérisé en ce qu'on isomérise un dérivé d'isoquinoléine de formule générale

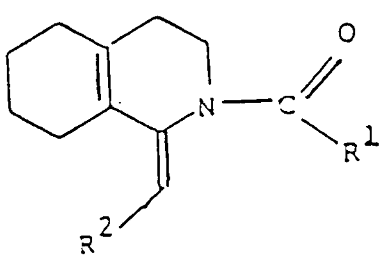

II

forme E

ou

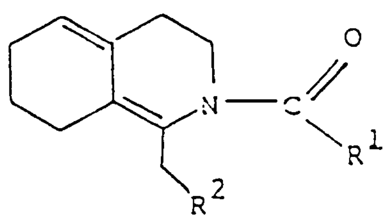

III

ou un mélange de dérivés d'isoquinoléine de formules générales

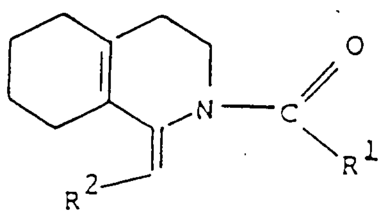

forme E II

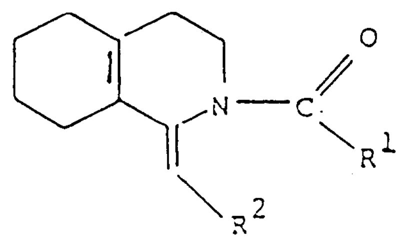

Forme Z I

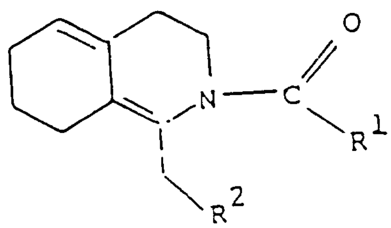

III

où $R^1$ représente un alkyle inférieur, un aryle ou un aryl-(alkyle inférieur), et $R^2$ représente un phényle ou un phényle para-méthoxy-substitué,
et que, si c'est souhaité, on hydrogène asymétriquement un dérivé d'isoquinoléine ainsi obtenu de formule générale suivante sous la forme Z

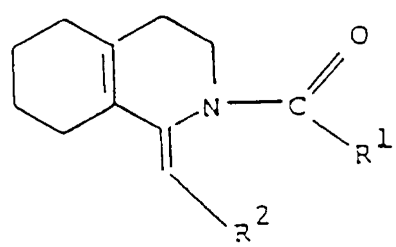

Forme Z I

où $R^1$ et $R^2$ ont la signification donnée ci-dessus, pour donner un composé de formule générale

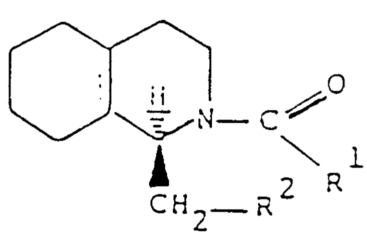

IV

où $R^1$ er $R^2$ ont la signification donnée ci-dessus.

**2.** Procédé selon la revendication 1, caractérisé en ce qu'on réalise l'isomérisation d'un composé de formule II sous forme E, d'un composé de formule III, ou d'un mélange de dérivés d'isoquinoléine de formules II (forme E), I (forme Z) et III par chauffage dans un hydrocarbure chloré ou dans un solvant contenant un hydrocarbure chloré ou par voie catalytique.

3. Procédé selon la revendication 1 ou 2, caractérisé en ce qu'on réalise l'isomérisation par chauffage à une température d'environ 50 °C à environ 200 °C, de préférence à une température de reflux pouvant atteindre 150 °C.

4. Procédé selon l'une des revendications 1 à 3, caractérisé en ce qu'on utilise comme hydrocarbure chloré lors de l'isomérisation du chlorure de méthylène, du chloroforme, ou du 1,2-dichloroéthane.

5. Procédé selon l'une des revendications 1 à 4, caractérisé en ce qu'on utilise comme solvant lors de l'isomérisation un alcool inférieur de 1 à 5 atomes de carbone.

6. Procédé selon la revendication 1, caractérisé en ce qu'on utilise comme catalyseur pour l'isomérisation catalytique un acide, notamment un acide halohydrique, l'iode ou un complexe de métal de transition.

7. Procédé selon la revendication 1, caractérisé en ce qu'on réalise l'hydrogénation asymétrique d'un composé de formule I en la forme Z, en présence d'un catalyseur de ruthénium de formule V ou Va

$Ru(Z)_2L$ V

ou

$[Ru\ (Z^2)_{2-n}(L)(X)]Z^3_n$ Va

où Z représente un halogène ou un reste de formule A-COO⁻, A est un alkyle inférieur, un aryle, un alkyle halogéné inférieur ou un aryle halogéné, $Z^2$ représente un halogène, X représente un benzène, hexaméthylbenzène, ou p-cymol et $Z^3$ représente un halogène, $BF_4$ $ClO_4$ ou $B(phényl)_4$ et n le nombre 1 ou 2, et L représente un ligand de formule VI ou VII :

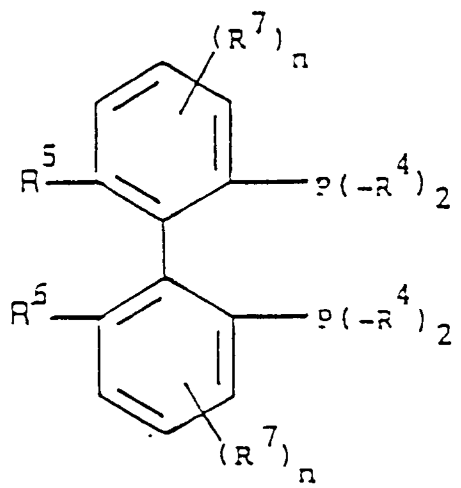

VI

dans laquelle $R^4$ représente un phényle, $R^5$ et $R^6$, qui peuvent être identiques ou différents, un hydrogène, un alkyle inférieur un alcoxy inférieur, un di(alkyle inférieur)amino, un hydroxyméthyle protégé ou $R^5$ et $R^6$ forment ensemble les groupes

$(-CH_2-)_m$, $-CH_2-O-CH_2-$, 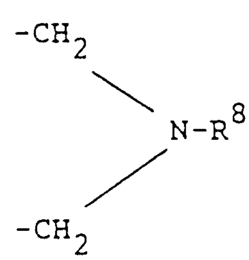 ou 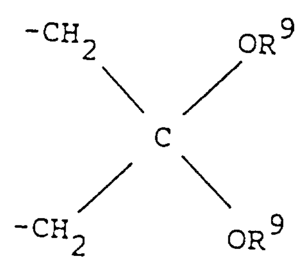

où m représente un nombre de 3 à 5, $R^8$ un alkyle inférieur, un phényle ou un benzyle et $R^9$ un alkyle inférieur ou les deux $R^9$ forment ensemble un di- ou triméthylène, $R^7$ représente un méthyle, alcoxy inférieur, di(alkyle inférieur)amino ou un fluor et n représente le nombre 0, 1, 2 ou 3,

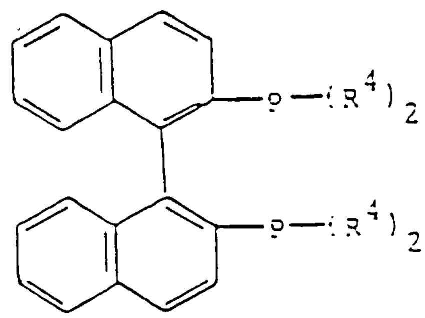

VII

où $R^4$ a la signification donnée ci-dessus, et les cycles naphtalène peuvent être substitués le cas échéant en position ortho par un méthyle, un éthyle, un halogène, un di(alkyle inférieur)amino ou un alcoxy inférieur.

**8.** Procédé selon la revendication 7, caractérisé en ce qu'on réalise l'hydrogénation asymétrique à une température d'environ 50 ° C à environ 200 ° C, de préférence entre environ 80 ° C et environ 160 ° C.